(19) Europäisches Patentamt
European Patent Office
Office européen des brevets

(11) Veröffentlichungsnummer: **0 252 351 B1**

(12) **EUROPÄISCHE PATENTSCHRIFT**

(45) Veröffentlichungstag der Patentschrift: **12.09.90**

(51) Int. Cl.[5]: **C07C 217/84**, A61K 7/13

(21) Anmeldenummer: **87108854.8**

(22) Anmeldetag: **20.06.87**

(54) **Neue 5-Alkoxy-2,4-diamino-alkylbenzole sowie Haarfärbemittel mit 5-Alkoxy-2,4-diamino-alkyl-benzolen.**

(30) Priorität: **07.07.86 DE 3622784**

(43) Veröffentlichungstag der Anmeldung: **13.01.88 Patentblatt 88/2**

(45) Bekanntmachung des Hinweises auf die Patenterteilung: **12.09.90 Patentblatt 90/37**

(84) Benannte Vertragsstaaten: **AT BE CH DE ES FR GB GR IT LI LU NL SE**

(56) Entgegenhaltungen:
**DE-A-2 628 999**
**DE-B-1 619 614**
**FR-A-2 542 193**
**GB-A-1 344 796**
**GB-A-2 163 154**
**US-A-2 381 877**

**CHEMICAL ABSTRACTS, Band 77, Nr. 5, 31. Juli 1972, Seite 450, Zusammenfassungsnr. 33688y, Columbus, Ohio, US; J.F. CORBETT "Benzoquinone imines. X. Mechanism and kinetics of the reactions of p-benzoquinone diimine and p-benzoquinone monoimine with C-methoxy-m-diamines and p-methoxy- and p-chlorophenols"trength fibre, e.g. glass fibre" CHEM. 1977, 000**

(73) Patentinhaber: **Wella Aktiengesellschaft, Berliner Allee 65, D-6100 Darmstadt(DE)**

(72) Erfinder: **Clausen, Thomas, Dr., Ernst-Pasqué-Strasse 35 A, D-6146 Alsbach(DE)**
Erfinder: **Konrad, Eugen, Mecklenburger Strasse 101, D-6100 Darmstadt(DE)**

(56) Entgegenhaltungen: (Fortsetzung)
**Die Akte enthält technische Angaben, die nach dem Eingang der Anmeldung eingereicht wurden und die nicht in dieser Patentschrift enthalten sind.**

**EP 0 252 351 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der Erfindung sind neue 5-Alkoxy-2,4-diamino-alkylbenzole und Mittel zur oxidativen Färbung von Haaren auf der Basis von bekannten Entwicklersubstanzen und 5-Alkoxy-2,4-diamino-alkylbenzolen als Kupplersubstanzen.

Für die Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels.

Die bevorzugt verwendeten Kupplersubstanzen sind m-Phenylendiamin und dessen Derivate, wie zum Beispiel 2,4-Diamino-anisol, 2,4-Diamino-phenetol, 2,4-Diamino-phenoxyethanol, 3,5-Diamino-2,6-dimethoxy-pyridin und 2-Amino-4(2'-hydroxyethyl)amino-anisol als Blaukuppler, 1-Naphthol, m-Aminophenol, 3-Amino-5-hydroxy-2,6-dimethoxy-pyridin und 5-Amino-o-kresol als Rotkuppler und Resorcin, 2-Methyl-resorcin und 4-Chlor-resorcin als Kuppler für den Braun-Blond-Bereich.

Als Entwicklersubstanzen werden bevorzugt 2,5-Diamino-toluol, 4-Amino-phenol und 1,4-Diamino-benzol verwendet, jedoch haben auch 2,5-Diamino-anisol, 2,5-Diamino-benzyl-alkohol und 2-(2'-Hydroxyethyl)-1,4-diamino-benzol eine gewisse Bedeutung erlangt. In bestimmten Fällen kann auch Tetraaminopyrimidin als Entwicklersubstanz eingesetzt werden.

An Oxidationsfarbstoffe, die zum Färben von menschlichen Haaren Verwendung finden, sind zahlreiche besondere Anforderungen gestellt. So müssen sie in toxikologischer und dermatologischer Hinsicht unbedenklich sein und die Erzielung von Färbungen in der gewünschten Intensität ermöglichen. Weiterhin ist es erforderlich, daß durch Kombination geeigneter Entwickler- und Kupplersubstanzen eine breite Palette unterschiedlicher Farbnuancen erzeugt werden kann. Außerdem wird für die erzielbaren Haarfärbungen eine gute Licht-, Dauerwell-, Säure- und Reibechtheit gefordert. Auf jeden Fall aber müssen solche Haarfärbungen ohne Einwirkung von Licht, chemischen Mitteln und Reibung über einen Zeitraum von mindestens 4 bis 6 Wochen stabil bleiben.

Die zur Zeit in Haarfärbemitteln verwendeten Kombinationen aus den oben genannten Entwickler- und Kupplersubstanzen erfüllen zwar weitgehend die genannten Anforderungen in Bezug auf die anwendungstechnischen Eigenschaften, sind aber nicht in der Lage, die Ansprüche bezüglich der toxikologischen Eigenschaften zu befriedigen. Insbesondere wird auch verlangt, daß die in Haarfärbemitteln verwendeten Verbindungen nicht oder nur wenig mutagen in Testsystemen wie denen von B.N. Ames sind.

So ist das 2,4-Diamino-toluol, welches gute färberische Eigenschaften besitzt, wegen seiner mutagenen Wirkung in vielen Ländern als Haarfarbstoff verboten.

Ein Rotstich, wie er mit der bekannten Kupplersubstanz 2-Amino-4-(2'-hydroxyethyl)amino-anisol erhalten wird, erschwert die Nuancierung von Farbtönen, weil der Rotanteil, der neben dem gewünschten Blau erhalten wird, durch den Zusatz weiterer Farbkomponenten kompensiert werden muß Die dabei auftretende Verschiebung der Tontiefe muß zusätzlich berücksichtigt werden.

Ebenso problematisch ist ein Grünstich, der sich zum Beispiel bei der Ausfärbung mit dem 3,5-Diamino-2,6-dimethoxypyridin ergibt. Ein solcher Grünstich erschwert die Herstellung von Aschtönen, bei denen ein bläulicher Überschein erwünscht ist. Die Kompensation der grünlichen Färbung erfordert ebenfalls den Zusatz weitere Farbkomponenten, wodurch wiederum die Nuancierung heller Farbtöne erschwert wird.

Das aus der eigenen DE-OS 3 430 513 bekannte 2,4-Diamino-5-tetrafluorethoxy-toluol zeigt zwar im Amestest keine mutagene Wirkung, jedoch sind Farbtiefe und Lichtechtheit der mit dieser Verbindung erzielbaren Färbungen nicht völlig zufriedenstellend. Zudem gelingt die Reinherstellung des 2,4-Diamino-5-tetrafluorethoxy-toluol nur durch aufwendige chromatographische Trennungen im Gramm-Maßstab, wodurch Haarfärbemittel mit einem Gehalt an 2,4-Diamino-5-tetrafluorethoxy-toluol stark verteuert werden.

Aus der DE-A 2 628 999 sind bestimmte 2-Alkoxy-3,5-amino-alkylbenzole bekannt. Diese ergeben in Kombination mit üblichen Entwicklersubstanzen, beispielsweise p-Toluylen-diamin, jedoch lediglich braune bis rotbraune Haarfärbungen.

Das in Chemical Abstracts, Band 77, Nr. 5, 31.07.1972, Seite 450, Nr. 33 688y beschriebene 2,4-Diamino-5-ethoxy-toluol ermöglicht zwar in Kombination mit üblichen Entwicklersubstanzen blaue Haarfärbungen, ist jedoch physiologisch sowie hinsichtlich der Lichtechtheit nicht völlig zufriedenstellend.

Aufgabe der Erfindung ist es daher, ein Haarfärbemittel auf der Basis neuer Kupplersubstanzen zur Verfügung zu stellen, welche einfach und billig herstellbar sind, in Kombination mit geeigneten Entwicklersubstanzen eine reine blaue Ausfärbung von großer Farbtiefe und Lichtechtheit ergeben sowie physiologisch günstige Eigenschaften aufweisen.

Hierzu wurde nun überraschend gefunden, daß sich die toxikologischen Eigenschaften von bestimmten üblicherweise in Oxidationshaarfärbemitteln angewandten Kupplersubstanzen durch die Einführung von verschiedenen Alkoxygruppen in diese Verbindungen wesentlich verbessern lassen.

Gegenstand der Erfindung sind daher neue 5-Alkoxy-2,4-diamino-alkylbenzole der allgemeinen Formel (I)

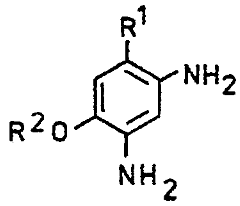

(I),

worin $R^1$ Methyl oder Ethyl darstellt und $R^2$ die Bedeutung Alkylrest mit 2 bis 4 Kohlenstoffatomen, Monohydroxyalkylrest mit 2 bis 4 Kohlenstoffatomen oder Dihydroxyalkylrest mit 3 bis 4 Kohlenstoffatomen hat, sowie deren Säureadditionssalz.

Als Beispiele für die neuen Kupplersubstanzen der Formel (I) seien 2,4-Diamino-5-(2'-hydroxyethyl)oxy-toluol und 2,4-Diamino-5-ethoxy-toluol genannt.

Die Herstellung von repräsentativen Vertretern der neuen Kupplersubstanzen ist in den Beispielen beschrieben. Allgemein lassen sich die Verbindungen auf zwei Wegen herstellen:

1. 5-Chlor-2,4-dinitro-toluol (M. Qvist und M. Moilanen, Acta Acad. Aboenis, Math. Phys., 14 (1943); (C.A. 38, 5491 (1944)) wird analog der Vorschrift von J. F. Corbett, J. Chem. Soc. Perkin II, 999 (1972) mit dem entsprechenden Alkohol, in dem vorher Kaliumhydroxid gelöst wurde, umgesetzt.

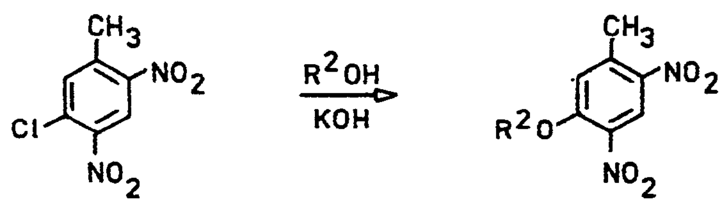


(II) (III)

Die katalytische Reduktion von (III) ergibt die Kupplersubstanz der allgemeinen Formel (I) mit $R^1 = CH_3$.

2. 2,4-Dinitro-5-alkyl-phenol wird an der Hydroxygruppe alkyliert. Zum Beispiel läßt sich das 2,4-Dinitro-5-methyl-phenol (IV) (J. R. Gibbs und P. W. Robertson, J. Chem. Soc. (London) 105, 1885-1892) durch Umsetzung mit einem gegebenenfalls substituierten Alkylhalogenid in die Vorstufe (III) überführen, deren katalytische Reduktion wie oben (I) ergibt.

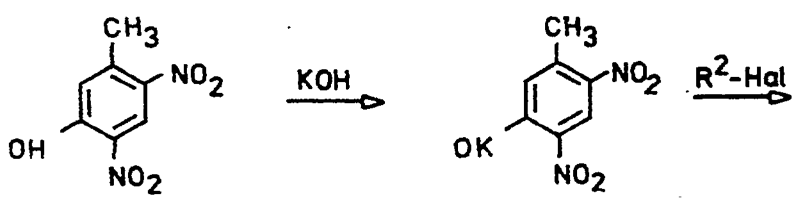


(III)

(IV)

Die Herstellung der Verbindungen der Formel (I) durch Alkylierung der Phenolvorstufe oder durch nucleophile Substitution der Chlorbenzolvorstufe ist in einfacher und somit billiger Weise in technischem Maßstab durchführbar. Die Säureadditionssalze der Verbindungen der Formel (I) sind durch Umsetzung mit der entsprechenden organischen oder anorganischen Säure erhältlich.

Man erhält eine neue Klasse von Kupplersubstanzen, mit physiologisch günstigen Eigenschaften, die entweder keine oder nur geringe Mutagenität aufweisen. Dies ist außerordentlich überraschend, da das 2,4-Diamino-toluol, das die zusätzliche Alkoxygruppe nicht enthält, ein bekanntes und sehr starkes Mutagen darstellt, so daß diese Verbindung sogar in dem Mutagenitätstest nach B.N. Ames (B.N. Ames, J. MacCann und E. Yamasaki, Mut. Res. 31, 347-363 (1975)) als Positivstandard eingesetzt werden kann, um die Wirksamkeit des Testsystems zu überprüfen.

Ein weiterer Vorteil der Kupplersubstanzen der allgemeinen Formel (I) ist der Farbton der Ausfärbung. Unabhängig von dem Rest $R^2$ wird zum Beispiel bei der Ausfärbung mit der üblichen Entwicklersubstanz 2,5-Diamino-toluol jeweils ein reines kaltes Blau ohne störende Rot- oder Grünanteile erhalten.

Weiterhin besitzen die mit den neuen Kupplersubstanzen erzielbaren Färbungen eine hohe Farbtiefe und eine unerwartet hohe Lichtechtheit.

Gegenstand der vorliegenden Erfindung ist ferner ein Mittel zur oxidativen Färbung von Haaren auf der Basis einer Entwicklersubstanz-Kupplersubstanz-Kombination, welches als Kupplersubstanz mindestens ein 5-Alkoxy-2,4-diaminoalkylbenzol der allgemeinen Formel (I) enthält.

Die Kupplersubstanzen der Formel (I) sollen in den Haarfärbemitteln entweder als freie Base oder in Form ihrer physiologisch verträglichen Salze mit anorganischen oder organischen Säuren, zum Beispiel als Chlorid, Sulfat, Phosphat, Acetat, Propionat, Lactat oder Citrat, eingesetzt werden. Die Verbindungen der Formel (I) sind gut in Wasser löslich. Sie weisen außerdem eine ausgezeichnete Lagerstabilität, insbesondere als Bestandteil der hier beschriebenen Haarfärbemittel, auf.

In den Haarfärbemitteln sollen die Kupplersubstanzen der Formel (I) in einer Konzentration von 0,01 bis 3,0 Gew.-%, vorzugsweise 0,1 bis 2,0 Gew.-%, enthalten sein.

Die Kupplersubstanz der Formel (I) wird im allgemeinen in etwa molarer Menge, bezogen auf die verwendeten Entwicklersubstanzen, eingesetzt. Wenn sich auch der äquimolare Einsatz als zweckmäßig erweist, so ist es doch nicht nachteilig, wenn die Kupplersubstanz in einem gewissen Überschuß oder Unterschuß zum Einsatz kommt. Es ist ferner nicht notwendig, daß die Entwicklerkomponente und die Kupplerkomponente einheitliche Produkte darstellen, vielmehr kann sowohl die Entwicklerkomponente ein Gemisch von bekannten Entwicklersubstanzen als auch die Kupplerkomponente ein Gemisch der erfindungsgemäßen Verbindung mit bekannten Kupplersubstanzen darstellen. Außerdem können in den Haarfärbemitteln zusätzlich bekannte Kupplersubstanzen, insbesondere Resorcin, 4-Chlor-resorcin, 2-Methyl-resorcin, 2-Amino-4-(2'-hydroxyethyl)amino-anisol, 2,4-Diamino-phenylethanol, 2,4-Diamino-phenoxyethanol, 1,5-Dihydroxy-tetralin, m-Amino-phenol, 3-Amino-2-methyl-phenol, 3-Amino-6-methylphenol, 4-Hydroxy-1,2-methylendioxy-benzol, 4-Amino-1,2-methylendioxy-benzol, 2,4-Diamino-anisol und 2,4-Diamino-phenetol enthalten sein.

Von den bekannten Entwicklersubstanzen kommen als Bestandteile der erfindungsgemäßen Haarfärbemittel vor allem 1,4-Diamino-benzol, 2,5-Diamino-toluol, 2,5-Diamino-anisol, 2,5-Diamino-benzylalkohol, 3-Methyl-4-amino-phenol und 4-Amino-phenol in Betracht.

Die Gesamtmenge der in den hier beschreibenen Haarfärbemitteln enthaltenen Entwicklersubstanz-Kupplersubstanz-Kombination soll etwa 0,1 bis 5,0 Gew.-%, vorzugsweise 0,5 bis 3,0 Gew.-%, betragen.

Zur Erzielung gewisser Farbnuancen können ferner auch übliche direktziehende Farbstoffe, beispielsweise Triphenylmethanfarbstoffe wie Diamond Fuchsine (C.I. 42 510) und Leather Ruby HF (C.I. 42 520), aromatische Nitrofarbstoffe wie 2-Amino-4,6-dinitro-phenol, 2-Nitro-1,4-diamino-benzol, 2-Amino-4-nitro-phenol und 2-Amino-5-nitrophenol, Azofarbstoffe wie Acid Brown 4 (C.I. 14 805) sowie Anthrachinonfarbstoffe wie 1,4-Diamino-anthrachinon enthalten sein.

Die neuen Haarfärbemittel können weiterhin auch mit sich selbst kuppelnde Farbvorstufen, wie zum Beispiel 2-Amino-5-methyl-phenol, 2-Amino-6-methyl-phenol, 2-Amino-5-ethoxy-phenol oder auch 2-Propylamino-5-amino-pyridin, enthalten.

Selbstverständlich können die Kuppler- und Entwicklersubstanzen sowie auch andere Farbkomponenten, sofern es Basen sind, in Form der physiologisch verträglichen Säureadditionssalze, wie beispielsweise als Hydrochlorid, Sulfat, Acetat und Lactat oder - sofern sie aromatische OH-Gruppen besitzen - in Form der Salze mit Basen, zum Beispiel als Alkaliphenolate, eingesetzt werden.Darüber hinaus können in den Haarfärbemitteln noch weitere übliche kosmetische Zusätze, beispielsweise Antioxidantien wie Ascorbinsäure oder Natriumsulfit, Parfümöle, Komplexbildner, Netzmittel, Emulgatoren, Verdicker, Pflegestoffe und andere vorhanden sein.

Die Zubereitungsform kann beispielsweise eine Lösung, insbesondere eine wäßrige oder wäßrig-alkoholische Lösung, sein. Die besonders bevorzugten Zubereitungsformen sind jedoch eine Creme, ein Gel oder eine Emulsion.

Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen snd zum Beispiel Lösungsmittel wie Wasser, niedere aliphatische Alkohole, beispielsweise Ethanol, Propanol und Isopropanol, sowie mehrwertige Alkohole wie Ethylenglykol, 1,2-Propylenglykol und Glycerin, weiterhin Netzmittel oder Emulgatoren aus den Klassen der anionischen, kationischen, amphoteren oder nichtionogenen oberflächenaktiven Substanzen wie Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Bentonit, Stärke, Polyacrylsäure, Cellulosederivate, Alginate, Vaseline, Paraffinöl und Fettsäuren sowie außerdem Pflegestoffe wie Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gew.-%, während die Verdicker in einer Menge von etwa 0,1 bis 25 Gew.-% in den Zubereitungen enthalten sein können.

Je nach Zusammensetzung können die erfindungsgemäßen Haarfärbemittel schwach sauer, neutral oder alkalisch reagieren. Insbesondere weisen sie einen pH-Wert im alkalischen Bereich zwischen 8,0 und 11,5 auf, wobei die Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid, Verwendung finden.

Für die Anwendung zur oxidativen Färbung von Haaren vermischt man die vorstehend beschriebenen Haarfärbemittel unmittelbar vor dem Gebrauch mit einem Oxidationsmittel und trägt eine für die Haarfärbebehandlung ausreichende Menge, je nach Haarfülle, im allgemeinen etwa 60 bis 200 g, dieses Gemisches auf das Haar auf. Als Oxidationsmittel zur Entwicklung der Haarfärbung kommt hauptsächlich Hydrogenperoxid, beispielsweise als 6-prozentige, wäßrige Lösung beziehungsweise dessen Additionsverbindungen an Harnstoff, Melamin oder Natriumborat, in Betracht. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet. Gegebenenfalls wird im Anschluß an diese Spülung mit einem Shampoo gewaschen und eventuell mit einer schwachen, physiologisch verträglichen, organischen Säure, wie zum Beispiel Zitronensäure oder Weinsäure, nachgespült.

Hinsichtlich der färberischen Möglichkeit bieten die erfindungsgemäßen Haarfärbemittel je nach Art und Zusammensetzung der Farbkomponenten eine breite Palette verschiedener Farbnuancen, welche sich von blonden über braune, aschige, matte, goldene bis hin zu blauen Farbtönen erstreckt. Hierbei zeichnen sich die Farbtöne durch gute Farbintensität und ausreichende Lichtechtheit aus.

**Herstellungsbeispiele**

**Beispiel 1:** 2,4-Diamino-5-ethoxy-toluol-dihydrochlorid

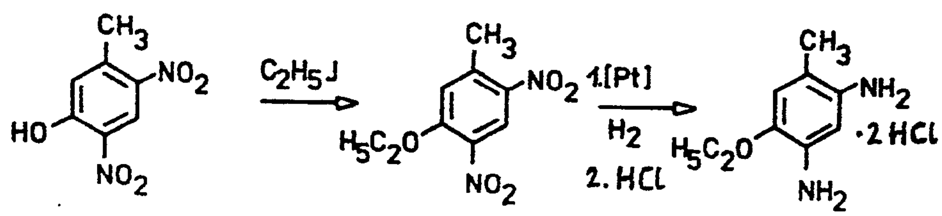


Stufe 1: 2,4-Dinitro-5-ethoxy-toluol

2,5 g (12,5 mmol) 2,4-Dinitro-5-methyl-phenol (I.R. Gibbs und P.W. Robertson, J. Chem. Soc. (London) 105, 1885) werden in 12,5 ml Dioxan gelöst, mit 1,5 ml (19 mmol) 2-Jod-ethan und 3,5 ml (25 mmol) Triethylamin versetzt und 24 Stunden lang auf 80 Grad Celsius erwärmt. Anschließend wird das Reaktionsgemisch auf Eis gegossen. Die ausgefallenen gelben Kristalle werden durch Absaugen isoliert. Man erhält 2,2 g (77 % der Theorie) der Ethoxyverbindung vom Schmelzpunkt 92 - 93 Grad Celsius.

NMR-Spektrum (Lösungsmittel $CDCl_3$):
8,67 (s, 3-H), 6,97 (s, 6-H), 4,30 (q, -O-$CH_2$-, J = 7 Hz), 2,72 (s, $CH_3$), 1,53 (t, O-$CH_2$$\underline{CH_3}$, J = 7 Hz)

Für dieses und alle folgenden NMR-Spektren gilt:

Gerät: Varian XL 100, 100 MHz-Spektrum;
s = Singulett, d = Dublett, t = Triplett, q = Quartett, m = Multiplett
Innerer Standard: Tetramethylsilan mit $delta_{TMS}$ = 0 ppm

Stufe 2: 2,4-Diamino-5-ethoxy-toluol-dihydrochlorid

Die Nitroverbindung der Stufe 1 wird in Ethanol gelöst und mit Wasserstoff an Platin katalytisch hydriert. Nach der Aufnahme der theoretischen Menge Wasserstoff wird vom Katalysator abfiltriert und die Lösung nach dem Einengen mit konzentrierter wäßriger Salzsäurelösung versetzt. Das entstandene Dihydrochlorid des 2,4-Diamino-5-ethoxy-toluol wird abgesaugt und getrocknet. Es zersetzt sich bei 210 Grad Celsius.

**Beispiel 2:** 2,4-Diamino-5-(2′-hydroxyethyl)oxy-toluol-dihydrochlorid

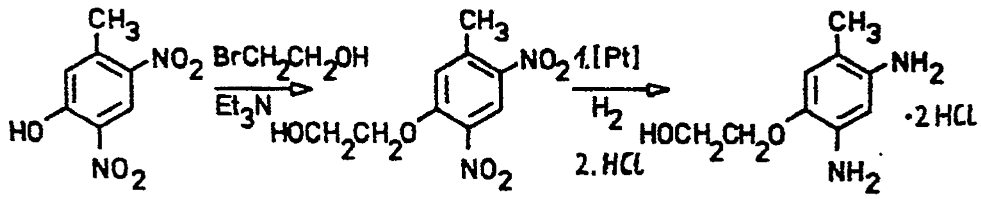


Stufe 1: 2,4-Diamino-5-(2'-hydroxyethyl)oxy-toluol

1,0 g (5 mmol) 2,4-Dinitro-5-methyl-phenol (siehe Beispiel 1) werden in 5 ml Dioxan gelöst, mit 0,5 ml (7 mmol) 2-Brom-ethanol und 1,7 ml (12 mmol) Triethylamin versetzt und 36 Stunden lang auf 85 Grad Celsius erwärmt. Anschließend wird das Reaktionsgemisch auf Eis gegossen. Die gelben Kristalle werden durch Absaugen isoliert. Man erhält 0,6 g (49 % der Theorie) des Dinitrophenolethers vom Schmelzpunkt 100 Grad Celsius.

NMR-Spektrum (Lösungsmittel $CDCl_3$):
8,72 (s, 3-H), 7,02 (s, 6-H), 4,34 (t, $-CH_2-$, J = 4,5 Hz), 4,05 (t, $-CH_2-$, J = 4,5 Hz), 2,73 (s, $CH_3$), 2,5-1,7 (breit, OH; das Signal verschwindet beim Schütteln der Probe mit $D_2O$).

Massenspektrum:
242 ($M^+$, 22 %), 198 (10 %), 182 (25 %), 181 (84 %), 165 (10 %), 135 (16 %), 109 (16 %), 107 (17 %), 73 (37 %), 46 (100 %).
Angabe in m/e (relative Intensität).

Stufe 2: 2,4-Diamino-5-(2'-hydroxyethyl)oxy-toluol

Die katalytische Reduktion der Nitroverbindung der Stufe 1 analog Beispiel 1 ergibt die Diaminoverbindung als Dihydrochlorid, das sich ohne zu schmeizen ab 45 °C zersetzt.

NMR-Spektrum (Lösungsmittel DMSO-$d_6$):
7,62 (s, 3-H), 7,18 (s, 6-H), 8,6-6,0 (sehr breit, $^+NH_3$; das Signal verschwindet beim Schütteln der Probe mit $D_2O$), 4,13 (t, $-CH_2-$, J = 4,5 Hz), 3,78 (t, $-CH_2-$, J = 4,5 Hz), 2,40 (s, $CH_3$).

Beispiele für Haarfärbemittel

| Beispiel 3: | Haarfärbemittel in Gelform |
|---|---|
| 0,10 g | 2,4-Diamino-5-(2'-hydroxyethyl)oxy-toluol-dihydrochlorid |
| 0,05 g | m-Aminophenol |
| 0,90 g | p-Toluylendiaminsulfat |
| 0,40 g | Resorcin |
| 0,30 g | Ascorbinsäure |
| 1,00 g | Hydroxyethylcellulose, hochviskos |
| 5,00 g | Lauryalkohol-diglykolethersulfat, Natriumsalz (28%-ige wäßrige Lösung) |
| 10,00 g | Ammoniak (22%-ige wäßrige Lösung) |
| 82,25 g | Wasser |
| 100,00 g | |

50 g des obigen Haarfärbemittels werden kurz vor dem Gebrauch mit 50 ml Hydrogenperoxidlösung (6-prozentig) vermischt und das Gemisch anschließend auf weiße menschliche Haare aufgetragen. Nach einer Einwirkungszeit von 30 Minuten bei 40 Grad Celsius werden die Haare mit Wasser gespült und getrocknet. Die Haare sind aschblond gefärbt.

| Beispiel 4: | Haarfärbemittel in Form einer wäßrigen Lösung |
|---|---|
| 0,15 g | 2,4-Diamino-5-ethoxy-toluol-dihydrochlorid |
| 0,80 g | 4-Amino-2-hydroxymethyl-phenol |
| 0,20 g | 5-Amino-2-methyl-phenol |
| 0,95 g | p-Toluylendiaminsulfat |
| 0,05 g | 2-Nitro-p-phenylendiamin |
| 0,02 g | 4-(2'-Ureidoethyl)amino-nitrobenzol |
| 0,15 g | Natriumsulfit |
| 10,00 g | Ethanol (96-prozentig) |
| 10,00 g | Ammoniak (25%-ige wäßrige Lösung) |
| 5,00 g | Laurylalkohol-diglykolethersulfat, Natriumsalz (28-prozentige wäßrige Lösung) |
| 72,68 g | Wasser |
| 100,00 g | |

25 g des obigen Haarfärbemittels werden kurz vor dem Gebrauch mit 25 ml Hydrogenperoxidlösung (6-prozentig) vermischt und das Gemisch anschließend auf weiße menschliche Haare aufgetragen. Nach einer Einwirkungszeit von 45 Minuten bei 35 Grad Celsius werden die Haare mit Wasser und einer verdünnten Zitronensäurelösung gespült und getrocknet. Die Haare sind in einem natürlichen Braunton eingefärbt.

| Beispiel 5: | Haarfärbelösung |
|---|---|
| 1,00 g | 2,4-Diamino-5-(2'-hydroxyethyl)oxy-toluol-dihydrochlorid |
| 3,00 g | 2-Ethyl-1,4-diamino-benzol-dihydrochlorid |
| 0,60 g | N-(2'-Hydroxyethyl)-3,4-methylendioxy-anilin |
| 0,50 g | 3-Amino-5-hydroxy-2,6-dimethoxy-pyridin-hydrochlorid |
| 0,10 g | m-Aminophenol |
| 10,00 g | Ammoniak (25-prozentige wäßrige Lösung) |
| 84,80 g | Wasser |
| 100,00 g | |

Man vermischt kurz vor dem Gebrauch 10 g des vorstehenden Haarfärbemittels mit 10 ml Hydrogenperoxidlösung (6-prozentig) und läßt die Mischung 30 Minuten lang bei 40 Grad Celsius auf blonde Naturhaare einwirken. Sodann werden die Haare mit Wasser gespült und getrocknet. Die Haare sind schwarz gefärbt.

Alle in der vorliegenden Patentanmeldung genannten Prozentzahlen stellen, soweit nicht anders genannt, Gewichtsprozente dar.

## Patentansprüche

1. 5-Alkoxy-2,4-diamino-alkylbenzol der allgemeinen Formel (I)

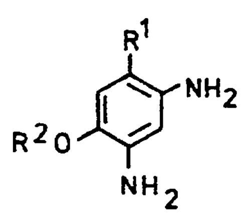

(I)

worin $R^1$ Methyl oder Ethyl darstellt und $R^2$ die Bedeutung Alkylrest mit 2 bis 4 Kohlenstoffatomen, Monohydroxyalkylrest mit 2 bis 4 Kohlenstoffatomen oder Dihydroxyalkylrest mit 3 bis 4 Kohlenstoffatomen hat, sowie dessen Säureadditionssalz.

2. 2,4-Diamino-5-(2'-hydroxyethyl)oxy-toluol.

3. 2,4-Diamino-5-ethoxy-toluol.

4. Mittel zur oxidativen Färbung von Haaren auf der Basis einer Entwicklersubstanz-Kupplersubstanz-Kombination, dadurch gekennzeichnet, daß es als Kupplersubstanz ein 5-Alkoxy-2,4-diamino-alkylbenzol der allgemeinen Formel (I)

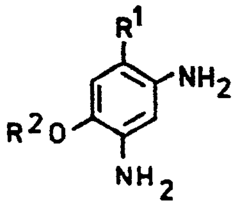

(I),

worin $R^1$ Methyl oder Ethyl darstellt und $R^2$ die Bedeutung Alkylrest mit 2 bis 4 Kohlenstoffatomen, Mono hydroxyalkylrest mit 2 bis 4 Kohlenstoffatomen oder Dihydroxyalkylrest mit 3 bis 4 Kohlenstoffatomen hat, oder dessen Säureadditionssalz enthält.

5. Mittel nach Anspruch 4, dadurch gekennzeichnet, daß $R^1$ Methyl darstellt.

6. Mittel nach Anspruch 4 und 5, dadurch gekennzeichnet, daß die Verbindung der Formel (I) ausgewählt ist aus 2,4-Diamino-5-(2'-hydroxyethyl)oxy-toluol und 2,4-Diamino-5-ethoxy-toluol.

7. Mittel nach Anspruch 4 bis 6, dadurch gekennzeichnet, daß die Kupplersubstanz der Formel (I) in einer Menge von 0,01 bis 3,0 Gew.-% enthalten ist.

8. Mittel nach Anspruch 4 bis 7, dadurch gekennzeichnet, daß die Entwicklersubstanz ausgewählt ist aus 1,4-Diamino-benzol, 2,5-Diamino-toluol, 2,5-Diamino-anisol, 2,5-Diamino-benzylalkohol, 2,5-Diamino-phenylethanol, 2-Methyl-4-amino-phenol, 3-Methyl-4-amino-phenol, 2-Aminomethyl-4-amino-phenol und 4-Amino-phenol.

9. Mittel nach Anspruch 4 bis 8, dadurch gekennzeichnet, daß es zusätzlich eine bekannte Kupplersubstanz enthält, welche ausgewählt ist aus Resorcin, 4-Chlor-resorcin, 2-Methyl-resorcin, 2-Amino-4-(2'-hydroxyethyl)amino-anisol, 2,4-Diamino-phenylethanol, 2,4-Diamino-phenoxyethanol, 2,4-Diamino-anisol, 2,4-Diaminophenetol, 1,5-Dihydroxy-tetralin, m-Aminophenol, 3-Amino-2-methyl-phenol, 3-Amino-6-methyl-phenol, 4-Hydroxy-1,2-methylendioxy-benzol, 4-Amino-1,2-methylendioxy-benzol, 4-(2'-Hydroxyethyl)amino-1,2-methylendioxy-benzol, 3,5-Diamino-2,6-dimethoxy-pyridin, 3,5-Diamino-2,6-Bis (2'-hydroxyethyl)oxy-pyridin.

## Revendications

1. 5-alcoxy-2,4-diamino-alcoylbenzène de formule générale (I)

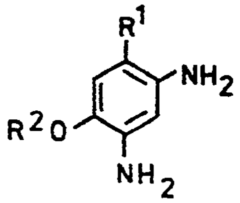

(I)

où $R_1$ représente méthyle ou éthyle et $R^2$ un radical alcoyle avec 2 à 4 atomes de carbone, un radical monohydroxyalcoyle avec 2 à 4 atomes de carbone ou un radical dihydroxyalcoyle avec 3 à 4 atomes de carbone, ainsi que leur sel d'addition acide.

2. 2,4-diamino-5-(2'-hydroxyéthyl)oxy-toluène.

3. 2,4-diamino-5-éthoxy-toluène.

4. Agent pour la teinture oxydative de cheveux basé sur une combinaison d'une substance de développement et d'une substance de couplage, caractérisé en ce qu'il comprend en tant que substance de couplage un 5-alcoxy-2,4-diamino-alcoylbenzène de formule générale (I)

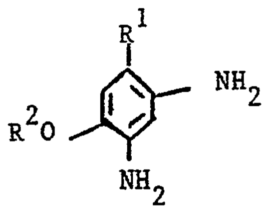

(I)

où $R^1$ représente méthyle ou éthyle et $R^2$ un radical alcoyle avec 2 à 4 atomes de carbone, un radical monohydroxyalcoyle avec 2 à 4 atomes de carbone ou un radical dihydroxyalcoyle avec 3 à 4 atomes de carbone, ou leur sel d'addition acide.

5. Agent selon la revendication 4, caractérisé en ce que $R^1$ représente méthyle.

6. Agent selon les revendications 4 et 5, caractérisé en ce que la liaison de la formule (I) est choisie parmi 2,4-diamino-5-(2'hydroxyéthyl)-oxy-toluène et 2,4-diamino-5-éthoxy-toluène.

7. Agent selon l'une des revendications 4 à 6, caractérisé en ce que la substance de couplage de formule (I) est contenue selon une quantité comprise entre 0,01 et 3.0% en poids.

8. Agent selon l'une des revendications 4 à 7, caractérisé en ce la substance de développement est choisie parmi 1,4-diamino-benzène, 2,5-diamino-toluène, 2,5-diamino-anisol, 2,5-diamino-benzylalcool, 2,5-diamino-phényléthanol, 2-méthyl-4-amino-phénol, 3-méthyl-4-amino-phénol, 2-aminométhyl-4-amino-phénol et 4-amino-phénol.

9. Agent selon l'une des revendications 4 à 8, caractérisé en ce comprend en outre une substance de couplage connue qui est choisie parmi résorcine, 4-chlore-résorcine, 2-méthyl-résorcine, 2-amino-4-(2'-hydroxyéthyl)amino-anisol, 2,4-diamino-phényléthanol, 2,4-diamino-phénoxyéthanol, 2,4-diamino-anisol, 2,4-diaminophénétol, 1,5-dihydroxy-tétraline, m-aminophénol, 3-amino-2-méthyl-phénol, 3-amino-6-méthyl-phénol, 4-hydroxy-1,2-méthylènedioxy-benzène, 4-amino-1,2-méthylènedioxy-benzène, 4-(2'-hydroxyéthyl)amino-1,2-méthylènedioxy-benzène, 3,5-diamino-2,6-diméthoxy-pyridine, 3,5-diamino-2,6-bis (2'-hydroxyéthyl)oxy-pyridine.

## Claims

1. 5-Alkoxy-2,4-diamino-alkylbenzene corresponding to the general formula (I)

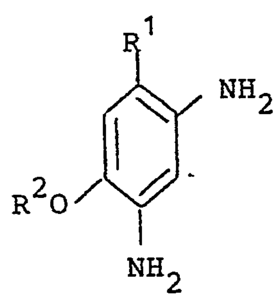

(I)

wherein $R^1$ denotes methyl or ethyl and $R^2$ denotes an alkyl group with 2 to 4 carbon atoms, a monohydroxyalkyl group with 2 to 4 carbon atoms or a dihydroxyalkyl group with 3 to 4 carbon atoms, and the acid addition salts thereof.

2. 2,4-Diamino-5-(2'-hydroxyethyl)oxy-toluene.

3. 2,4-Diamino-5-ethoxy-toluene.

4. Agent for the oxidative dyeing of hair based on a combination of developer substance and coupler substance, characterised in that the coupler substance contained therein is a 5-alkoxy-2,4-diamino-alkylbenzene corresponding to the general formula (I)

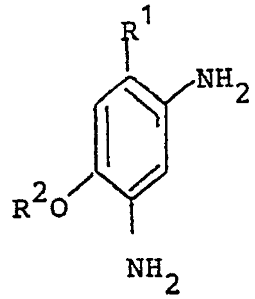

(I)

wherein $R^1$ denotes methyl or ethyl and $R^2$ denotes an alkyl group with 2 to 4 carbon atoms, a monohydroxyalkyl group with 2 to 4 carbon atoms or a dihydroxyalkyl group with 3 to 4 carbon atoms, or the acid addition salts thereof.

5. Agent according to claim 4, characterised in that $R^1$ denotes methyl.

6. Agent according to claims 4 and 5, characterised in that the compound of formula (I) is selected from 2,4-diamino-5-(2'-hydroxyethyl)oxy-toluene and 2,4-diamino-5-ethoxy-toluene.

7. Agent according to claims 4 to 6, characterised in that the coupler substance of formula (I) is contained therein in a quantity of from 0.01 to 3.0% by weight.

8. Agent according to claims 4 to 7, characterised in that the developer substance is selected from 1,4-diaminobenzene, 2,5-diamino-toluene, 2,5-diamino-anisole, 2,5-diamino-benzyl alcohol, 2,5-diaminophenylethanol, 2-methyl-4-amino-phenol, 3-methyl-4-amino-phenyl, 2-aminomethyl-4-amino-phenol and 4-amino-phenol.

9. Agent according to claims 4 to 8, characterised in that it contains, in addition, a known coupler substance selected from resorcinol, 4-chloro-resorcinol, 2-methylresorcinol, 2-amino-4-(2'-hydroxyethyl)amino-anisole, 2,4-diamino-phenylethanol, 2,4-diaminophenoxyethanol, 2,4-diamino-anisole, 2,4-diaminophenetole, 1,5-dihydroxytetraline, m-aminophenol, 3-amino-2-methyl-phenol, 3-amino-6-methylphenol, 4-hydroxy-1,2-methylenedioxybenzene, 4-amino-1, 2-methylenedioxy-benzene, 4-(2'-hydroxyethyl)amino-1, 2-methylenedioxy-benzene, 3,5-diamino-2,6-dimethoxy-pyridine and 3,5-diamino-2,6-bis-(2'-hydroxyethyl)oxy-pyridine.